Europäisches Patentamt

European Patent Office

Office européen des brevets

(11) Publication number: **0 164 086**
A1

# EUROPEAN PATENT APPLICATION

(21) Application number: 85106793.4

(22) Date of filing: 04.03.82

(51) Int. Cl.⁴: **A 61 F 7/00,** A 47 C 21/04

(30) Priority: 28.03.81 JP 44179/81
30.07.81 JP 118455/81
06.08.81 JP 122393/81
06.08.81 JP 122394/81
06.08.81 JP 122395/81
06.08.81 JP 122396/81

(43) Date of publication of application: 11.12.85
**Bulletin 85/50**

(84) Designated Contracting States: **DE FR GB IT NL**

(60) Publication number of the earlier application in accordance with Art. 76 EPC: 0061843

(71) Applicant: **Nakamatsu, Yoshiro, 1-10-309, 5-chome, Minami Aoyama Minato-ku, Tokyo (JP)**

(72) Inventor: **Nakamatsu, Yoshiro, 1-10-309, 5-chome, Minami Aoyama Minato-ku, Tokyo (JP)**

(74) Representative: **Shaw, Laurence, George House George Road, Edgbaston Birmingham B15 1PG (GB)**

(54) Apparatus for increasing brain activity.

(57) Apparatus for increasing the brain activity of a person lying horizontal includes heating means (324, 340) to heat the person's feet to 25 to 60°C, and cooling means (325, 325A, 370) to cool the persons head to 0°C to 20°C, and to cause a temperature difference of about 5 to 60°C between the feet and head.

EP 0 164 086 A1

0164086

APPARATUS FOR INCREASING
BRAIN ACTIVITY

The invention relates to apparatus for increasing the brain activity of a human person while that person is horizontal, e.g. lying on a bed or similar item of furniture.

It is known to apply extremes of heat or cold to various parts of the body of a person while the person is lying on a hospital operating table, for medical purposes e.g. to treat tumours etc.

The invention is based upon the discovery that if the head of a person lying generally horizontally is cooled at the same time as the feet are warmed, the mental activity of the person is increased.

The cooling and heating means may be separate or interconnected.  My investigations have shown that unless both cooling and heating take place at the same time, the brain activity is not increased, as the evaluations reported in the Description of my co-pending Application 82301097.0 (0061843), Agents Ref: 2077.  It is much preferred to cool the head to a temperature of about 0°C to

20°C, and to warm the feet to about 25°C to 60°C. In this way the temperature difference between the head and the feet can range from about 5°C to about 60°C.

The cooling element of the cooling means may be made of a sheet material reflecting alpha waves from the human brain back to the head of the person. The cooling element may be made of a polyester film having aluminium deposited on one side or a silicon rubber sheet containing aluminium powder. The alpha wave reflecting and cooling element may be assembled into a pillow which is used on a bed or mattress.

In order that the invention may be well understood, it will now be described, by way of example, with reference to the accompanying diagrammatic drawings in which:

Figure 1 is a side elevation view of a mattress of the invention and Figure 2 is a perspective view, partly in section, of apparatus shown in Figure 1;

Figure 3 is a side elevation view of another apparatus of the invention, and Figure 4 is a perspective view of the cooling member thereof;

Figure 5 is a front elevation of a pillow of the invention.

3

0164086

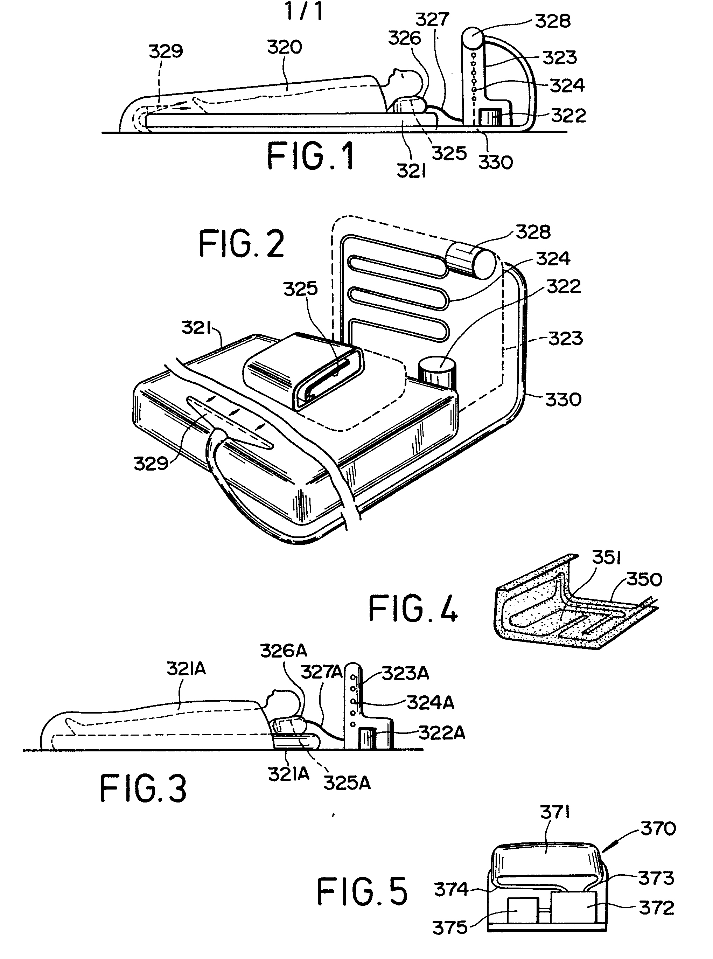

Figures 1 and 2 show the invention applied to a person 320 lying on a mattress 321 or a bed. A cooling and warming circuit comprises a compressor 322 received in a housing 323 and driven by any suitable means such as electric motor and a condenser 324 consisting of a convoluted heat-radiation pipe which receives a compressed coolant such as FREON from the compressor 322 also housed in the housing 323. An evaporator 325 is mounted in a pillow 326 and comprises a cooling element which receives the coolant from the condenser 324 through a capillary pipe 327. A blower 328 is mounted on the top of the housing 323 for feeding air heated by contacting with the condenser 324 to a nozzle 329 placed at the feet of the lying person 320 through a flexible hose 330. The person 320 lying on the mattress 321 will be cooled at his head and warmed at his feet. In a modification a thermoelement 340 is housed in a pillow 326 with a cold section disposed against a pillow portion which is engaged by the head of a person lying on a mattress. The hot section of the thermoelement is connected to a blower placed adjacent to the feet of the person lying on the mattress.

Figures 3 and 4 show an embodiment in which the cooling element or evaporator 325A is mounted in the pillow 326A and comprises a cooling plate 350 of thinner sheet material having a coolant passage 351. This cooling element provides

0164086

a soothing sound like the murmur of a brook which is produced when the coolant from the condenser 324A is evaporated at the evaporator 325A. A person 320A lying on the mattress 321A can be cooled at his head by the cooling element 325A and at the same time mentally calmed by the sound from the interior of the pillow 326A.

Figure 5 shows a pillow 370 comprising a head-engaging hollow portion 371, a pump 372 mounted in the pillow 370 and connected to the hollow portion 371 through pipe portions 373 and 374, and a motor 375 disposed in the pillow 370 for driving the pump 372. Liquid such as water is circulated from the pump 374 to the hollow portion 371 through the pipe portions 373 and 374 so that a person using this pillow 370 will be cooled at his head by water and at the same time mentally calmed by a sound like the murmur of a brook which is produced by the circulating water.

CLAIMS

1. Apparatus for treating a human person while the person is lying generally horizontal, the apparatus including means to apply heat and/or to cool selected parts of the body, characterised in that the heating means (324, 340) is arranged to heat the feet of the person (320, 320A) to a temperature of about 25°C to 60°C, and in that the cooling means (325, 325A, 370) is arranged to cool the head to about 0°C to 20°C, whereby a temperature difference in the range of about 5°C to about 60°C exists between the head and feet of the person (320, 320A).

2. Apparatus according to Claim 1, characteerised in that the cooling and warming means comprises a thermoelement utilising the Peltier effect with the cold section located in a head rest and the hot section located in a foot rest.

3. Apparatus according to Claim 1, characterised in that the cooling and warming means is mounted in a head pillow (326, 326A) and has a cold section (325, 350) to be engaged by the head of a person (320, 320A) using the pillow and a hot section (328), connected with blower (328) means via a conduit (330) and including a
nozzle (329) adapted to be placed adjacent to the
fee of the user (326).

4.  Apparatus according to Claim 1 or 3 <u>characterised</u>
    <u>in that</u> the cooling and warming means comprises a housing
    (323) containing a compressor (322) for compressing a
    coolant and a condenser (324) for receiving the compressed
    coolant and radiating heat, and blower means (328) for
    receiving air heated at the condenser (324) and feeding
    the heated air to another nozzle (329) via flexible duct
    means (330), and an evaporator (325) disposed in a head
    pillow (370) and connected with the condenser through
    a capillary pipe (327) for evaporating the coolant from
    the condenser (324).

5.  Apparatus according to Claim 1, <u>characterised in that</u>
    the evaporator comprises a cooling plate made of material
    adapted to reflect alpha waves from the human brain.

6.  Apparatus according to any preceding Claim, <u>characterised</u>
    <u>in that</u> the cooling element or evaporator (325A) is present
    in a pillow (326A) and adapted to produce a soothing noise.

7.  Apparatus according to Claim 6, <u>characterised in that</u>
    a pillow (370) includes a pump (372) to circulate a liquid
    through a hollow portion (371) to produce a mentally
    calming sound.

0164086

1/1

FIG.1

FIG.2

FIG.4

FIG.3

FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 195 421 (O.A. SMIRNOV et al.) <br> * Page 9, line 34 - page 10, line 9; figures * | 1 | A 61 F 7/00 <br> A 47 C 21/04 |
| | --- | | |
| A | DE-A-1 489 635 (M. VON ARDENNE) <br><br> * Claim 1; figures * | | |
| | --- | | |
| A | US-A-4 026 299 (J.W. SAUDER) | | |
| | --- | | |
| A | FR-A-1 566 614 (RAYNAUD et al.) | | |
| | --- | | |
| A | US-A-3 908 655 (H.B. LUND) | | TECHNICAL FIELDS SEARCHED (Int. Cl.4) |
| | --- | | |
| A | US-A-4 134 615 (J.F. JENKINS) | | A 61 F <br> A 47 C <br> A 61 B |
| | --- | | |
| A | US-A-4 306 747 (L.C. MOSS) | | |
| | --- | | |
| A | DE-A-1 956 352 (E. LIEBAU) | | |
| | --- | | |
| A | CH-A- 446 661 (H. KRANTZ) | | |
| | ----- | | |

The present search report has been drawn up for all claims

| Place of search <br> THE HAGUE | Date of completion of the search <br> 13-09-1985 | Examiner <br> STEENBAKKER J. |
|---|---|---|